(19) Europäisches Patentamt
European Patent Office
Office européen des brevets

(11) **EP 2 749 227 A1**

(12) **EUROPEAN PATENT APPLICATION**

(43) Date of publication:
**02.07.2014 Bulletin 2014/27**

(51) Int Cl.:
**A61B 8/00** (2006.01)   **A61B 8/08** (2006.01)
**A61B 5/00** (2006.01)

(21) Application number: **13196355.5**

(22) Date of filing: **10.12.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.12.2012 JP 2012286547**

(71) Applicant: **CANON KABUSHIKI KAISHA
Ohta-ku
Tokyo 146-8501 (JP)**

(72) Inventors:
• **Oyama, Kenji
  Tokyo, Tokyo 146-8501 (JP)**
• **Abe, Hiroshi
  Tokyo, Tokyo 146-8501 (JP)**

(74) Representative: **TBK
Bavariaring 4-6
80336 München (DE)**

(54) **Object information acquiring apparatus**

(57)   An object information acquiring apparatus is used that include a probe; a signal processor that generates a first image based on photoacoustic waves, which are generated from an object irradiated with light and which are received by the probe, and that generates a second image based on ultrasound waves, which are received by the probe and then transmitted to the object and reflected off the object; a controller that displays the first image and the second image on a display unit; a first corrector that corrects brightness of the first image; and a second corrector that corrects brightness of the second image independently from the first corrector.

FIG. 1

EP 2 749 227 A1

**Description**

BACKGROUND OF THE INVENTION

Field of the Invention

The present invention relates to an object information acquiring apparatus.

Description of the Related Art

(Photoacoustic tomography)

The technology of photoacoustic tomography (hereinafter referred to as "PAT") for acquiring functional information of a living body using light and ultrasound waves has been proposed. PAT is known to be particularly effective for the diagnosis of skin cancer and breast cancer, and is expected to become a medical appliance that will replace ultrasound imaging devices, X-ray devices and MRI devices which have been conventionally used for the foregoing diagnosis.

When a biological tissue is irradiated with pulsed light such as visible light or near infrared light, light absorbing materials within the living body; specifically, substances such as hemoglobin in the blood absorb the energy of the pulsed light and instantaneously swell and, consequently, photoacoustic waves (typically ultrasound waves) are generated. This phenomenon is referred to as the photoacoustic effect, and PAT visualizes the information of the biological tissues by measuring the photoacoustic waves. As a result of visualizing the light energy absorption density distribution (density distribution of the light absorbing material in the living body which became the generation source of the photoacoustic waves) as the information of the biological tissues, active angiogenesis caused by cancer tissue can by imaged. Moreover, by utilizing the optical wavelength dependency of the generated photoacoustic waves, functional information such as the oxygen saturation of blood can be obtained.

In addition, with the PAT technology, since non-irradiation and noninvasive image diagnosis is enabled as a result of using light and ultrasound waves in the imaging of biological information, PAT has a significant advantage in terms of burden on patients. Accordingly, the utilization of PAT is expected in the screening and early diagnosis of breast cancer in substitute for X-ray devices in which repeated diagnosis is difficult.

With PAT, an initial sound pressure $P_o$ of the photoacoustic waves that are generated as a result of the light absorbing materials absorbing light based on the photoacoustic measurement principle is calculated according to Formula (1) below.

$$P_o = \Gamma \cdot \mu_a \cdot \Phi \qquad \ldots (1)$$

Here, $\Gamma$ is the Grueneisen coefficient, which is obtained by dividing the product of squares of the volume expansion coefficient $\beta$ and the speed of sound c by the specific heat at constant pressure $C_p$. It is known that F becomes an approximately constant value depending on the object, $\mu_a$ is the light absorption coefficient of the light absorbing material, and $\Phi$ is the amount of light within the object; that is, the amount of light (optical fluence) that actually reached the light absorbing material.

According to Formula (1), since the initial sound pressure $P_o$ is dependent on the product of the light absorption coefficient $\mu_a$ and the amount of light $\Phi$, even if the light absorption coefficient is a small value, the generated photoacoustic waves will be large if the amount of light is great. Moreover, even if the amount of light is a small value, the generated photoacoustic waves will also be large if the light absorption coefficient is great.

Note that, by dividing the initial sound pressure distribution $P_o$ by the Grueneisen coefficient $\Gamma$, it is possible to calculate the distribution of the product of $\mu_a$ and $\Phi$; that is, the light energy absorption density distribution. The initial sound pressure distribution $P_o$ can be obtained by measuring the time change of the sound pressure P of the photoacoustic waves that reach the probe upon propagating within the object.

In addition, in order to calculate the distribution of the light absorption coefficient $\mu_a$ in the object to be diagnosed, it is necessary to calculate the distribution of the amount of light $\Phi$ in the object. Since the measured light penetrates and reaches the deep part of the object while being strongly diffused and attenuated within the object, the amount of light $\Phi$ that actually reached the light absorbing material is calculated from the amount of optical attenuation and the depth of penetration in the object. When the surface region of the object is irradiated with pulsed light of a uniform amount of light, and the boundary conditions are set on the assumption that light in the object has propagated on the plane waves, the amount of light $\Phi$ in the object can be represented with Formula (2) below.

$$\Phi = \Phi_0 \cdot \exp(-\mu_{eff} \cdot d) \qquad \ldots (2)$$

Here, $\mu_{eff}$ is the average effective optical attenuation coefficient of the object, and $\Phi_0$ is the amount of light of the pulsed light with which the object was irradiated; that is, the amount of light on the object surface. Moreover, d is the distance from the light irradiation region of the object surface to the light absorbing material that generated the photoacoustic waves.

As described above, the light absorption coefficient distribution $\mu_a$ can be calculated from Formula (1) and the light energy absorption density distribution $\mu_a\Phi$.

(Ultrasound wave measurement)

An ultrasound measurement device transmits, to an object, an ultrasound beam that is formed by synthesizing a plurality of ultrasound waves with an ultrasound probe (hereinafter sometimes simply referred to as a "probe"). In addition, information of the tissues in the object is obtained by receiving an ultrasound echo that is reflected within the object. Moreover, by repeating ultrasound measurement while performing two-dimensional scanning of the object using the ultrasound beam, the form information of tissues in the object can be three-dimensionally measured and visualized.

Measurement using ultrasound waves is effective because, for instance, it is possible to detect the specificity of the tumor (for instance, difference of breast cancer, cystic tumor, solid matter or the like) in the diagnosis of breast cancer to be carried out by the breast oncology department, it is possible to detect acinous cancer, and it is possible to recognize the position and form of the tumor in the depth direction, and is being broadly used as a diagnostic device. Since an ultrasound diagnostic device can measure the biological tissues noninvasively through acoustic measurement using ultrasound waves, there is a significant advantage in terms of burden of patients. Thus, an ultrasound diagnostic device is utilized for the screening and early diagnosis of breast cancer in substitute for other diagnostic devices in which repeated diagnosis is difficult.

Generally speaking, with the diagnosis of breast cancer, a comprehensive malignancy diagnosis is performed based on the results of palpation or image diagnosis of a plurality of modalities. Japanese Patent No. 4448189 discloses, as technology for improving the accuracy of the diagnosis of breast cancer, technology of performing image diagnosis based on a plurality of modalities while
the state of the object is maintained consistently. According to this technology, it is possible to acquire a photoacoustic wave image and an ultrasound image which visualize the active angiogenesis caused by breast cancer based on the photoacoustic principle while the state of the object is maintaining consistently.

Moreover, Japanese Patent Application Laid-Open No. 2010-015535 discloses time gain control (TGC) of increasing the amplification grain according to the time required for the photoacoustic waves generated within the object to reach the probe upon giving consideration to the attenuation characteristics of the amount of light associated with the penetration of the pulsed light into the object. Based on TGC, it is possible to obtain a photoacoustic wave image having a uniform brightness level regardless of the measured depth.

However, previously, JOURNAL OF BIOMEDICAL OPTICS 1(3), 330-334 (JULY 1996) and other documents have reported that there is consideration variation in $\mu_{eff}$ in the breast depending on the object. Since the attenuation characteristics of the amount of light $\Phi$

in the object depends strongly on $\mu_{eff}$, there are cases where the difference in the amount of light becomes tenfold or greater even when compared with the same penetration depth due to the variation in $\mu_{eff}$, and the optimal correction value relative to the measured depth differs for each object.

Patent Literature 1: Japanese Patent No. 4448189
Patent Literature 2: Japanese Patent Application Laid-Open No. 2010-015535

Non Patent Literature 1 : JOURNAL OF BIOMEDICAL OPTICS 1(3), 330-334 (JULY 1996)

SUMMARY OF THE INVENTION

The present invention in its first aspect provides an object information acquiring apparatus as specified in claims 1 to 14.

Further features of the present invention will become apparent from the following description of exemplary embodiments with reference to the attached drawings.

BRIEF DESCRIPTION OF THE DRAWINGS

FIG. 1 is a schematic diagram of the apparatus configuration of the object information acquiring apparatus in Embodiment 1;
FIG. 2 is a flowchart showing the acquisition and correction of object information in Embodiment 1;
FIG. 3 is a conceptual diagram explaining the corrector of object information in Embodiment 1;
FIGS. 4A and 4B are conceptual diagrams explaining the correction method of object information in Embodiment 1;
FIG. 5 is a flowchart explaining the correction of object information in Embodiment 1;
FIG. 6 is a flowchart explaining the initial correction of object information in Embodiment 2;
FIG. 7 is a flowchart showing the acquisition and adjustment of object information in Embodiment 3;
FIG. 8 is a conceptual diagram explaining the corrector of object information in Embodiment 4; and
FIGS. 9A to 9C are conceptual diagrams explaining the correction method of object information in Embodiment 4.

DESCRIPTION OF THE EMBODIMENTS

In order to obtain a photoacoustic wave image having a uniform brightness level regardless of the measured depth, it is preferable to perform correction relative to the measured depth based on a combination of the attenuation characteristics of the amount of light associated with the penetration of the pulsed light into the object, and the attenuation characteristics until the photoacoustic waves generated in the object reach the probe. The attenuation characteristics of the amount of light in the object are represented by Formula (2), and the attenuation characteristics of the acoustic waves in the living body also generally indicate characteristics of an exponential function.

The same can be said in order to obtain an ultrasound image having a uniform brightness level regardless of the measured depth. In other words, it is preferable to perform correction relative to the measured depth in light of the attenuation characteristics of the ultrasound waves until they reach inside the object based on the transmission of such ultrasound waves, and the attenuation characteristics of the ultrasound waves until the ultrasound echoes reflected inside the object reach the probe.

In addition, in most cases, the attenuation characteristics of the amount of light and the attenuation characteristics of the photoacoustic waves and the ultrasound waves are not uniform inside the object. Thus, there are demands for improving the accuracy of measuring the distribution of each of the foregoing attenuation characteristics (that is, the attenuation coefficients).

Thus, for example, devices and methods for directly measuring the respective attenuation coefficients or operational methods for estimating the respective attenuation coefficients based on indirectly measurable information are being considered. Nevertheless, these devices and methods cannot be implemented easily since there are problems such as the diagnostic operation becoming complicated, or the devices and processing requiring high costs.

As described above, with photoacoustic wave images and ultrasound images, independent correction control needed to be performed on the respective images since there is a potential difference in the image characteristics in the depth direction caused by the difference in the attenuation characteristics of the respective measurement principles.

Moreover, in order to obtain a more easy-to-observe image, it was necessary to provide the user with a corrector for controlling the correction.

The present invention was devised in view of the foregoing problems, and this invention is developed to provide technology for performing correction control which can be adjusted by the user, independently for photoacoustic wave images and ultrasound images, in an object information acquiring apparatus which acquires photoacoustic wave images and ultrasound images.

The preferred embodiments of the present invention are now explained with reference to the appended drawings. However, the size, material, shape and relative arrangement of components described below are to be suitably changed depending on the configuration and various conditions of the apparatus to which the present invention is to be applied, and these embodiments are not intended to limit the scope of the present invention to the following descriptions.

<Embodiment 1>

The object information acquiring apparatus according to the present invention generates an image by visualizing object information based on photoacoustic waves and ultrasound waves. The object information acquiring apparatus of the present invention comprises a configuration for transmitting ultrasound waves to an object and receiving ultrasound echo signals, and a configuration for causing the object to be irradiated with light and receiving the generated photoacoustic wave signals. The correction of object information in this embodiment comprises a corrector of a brightness level in the depth direction relative to the generated image, and the brightness value of the image is corrected according to the operation of the corrector by the user. The characteristic portions of the present invention are now mainly explained.

Note that the object information that is generated based on ultrasound echo signals (hereinafter simply referred to as the "ultrasound signals") is information which reflects the difference in the acoustic impedance of the issues in the object. The object information that is generated based on the photoacoustic wave signals indicates the generation source distribution of the acoustic waves that are generated based on photo irradiation, the initial sound pressure distribution in the object, the light energy absorption density distribution derived from the initial sound pressure distribution, the absorption coefficient distribution, or the concentration information distribution of substances configuring the tissues. The concentration information distribution of substances is, for example, the oxygen saturation distribution or oxidized/deoxygenated hemoglobin concentration distribution or the like. As a result of appropriately processing the foregoing object information, it is possible to generate image data that can be displayed on a display.

Moreover, the acoustic waves referred to in the present invention are typically ultrasound waves, and include elasticity waves referred to as sound waves, ultrasound waves, and acoustic waves. In particular, the elasticity waves that are generated in the object when the inside of the object is irradiated with light such as near infrared rays are indicated as photoacoustic waves. The probe used in the present invention receives the acoustic waves of the ultrasound waves and the photoacoustic waves that were generated or reflected in the object.

(Apparatus configuration)

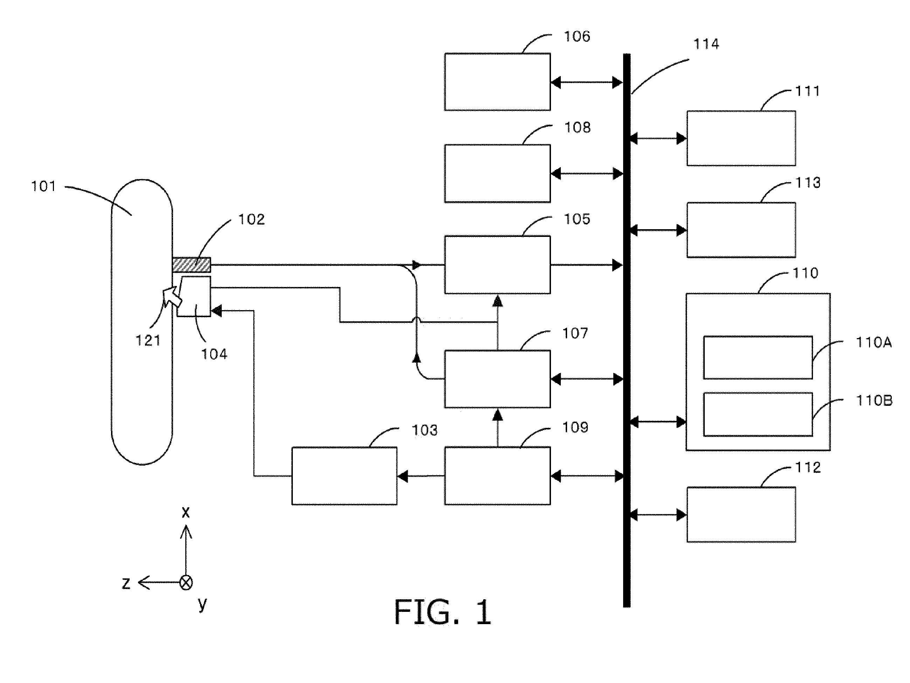

FIG. 1 is a schematic diagram of the apparatus configuration of the object information acquiring apparatus in this embodiment. An object 101 to be measured is, for example, a breast in the diagnosis of breast cancer to be carried out by the breast oncology department.

The object information acquiring apparatus comprises a probe 102 which sends and receives ultrasound waves and receives photoacoustic waves by coming into contact with the object 101, a light source 103 which generates light, and an irradiation optical system 104 which irradiates light with which the object 101 is irradiated. The apparatus additionally comprises a signal reception unit 105 which amplifies the signals detected by the probe 102 and converts the detected signals into digital signal, and a photoacoustic wave signal processor 106 which performs integration processing of the detected photoacoustic wave signals. The apparatus additionally comprises an ultrasound transmission controller 107 which applies ultrasound transmission drive signals to the probe 102, and an ultrasound signal processor 108 which performs receiving focus processing and the like based on the detected ultrasound signals. The apparatus additionally comprises an operation unit 110 which used by the user (mainly testers such as health care providers) to input commands to the apparatus for commencing measurement or parameters required for the measurement, and an image component 111 which configures photoacoustic wave images and ultrasound images, respectively, from the acquired photoacoustic wave signals and ultrasound signals. The apparatus additionally comprises a display unit 112 which displays the configured images and a user interface (UI) for operating the apparatus. The apparatus additionally comprises a control processor 109 which generates control information required for the measurement operation upon receiving various operations from the user via the operation unit 110 and controls the respective functions via a system bus 114, and a storage unit 113 which stores the acquired signals and the setting information related to the measurement operation.

The signal reception unit, the photoacoustic wave signal processor, the ultrasound signal processor and the image component correspond to the signal processor of the present invention with respect to the point that they generate images by performing various types of processing on the acoustic wave. The display unit corresponds to the display of the present invention.

(Probe)

The probe 102 is configured by arranging a plurality of acoustic elements. Photoacoustic wave signals can be obtained by these acoustic elements detecting the photoacoustic waves that are generated inside the object when the object is irradiated with light 121, and converting the generated photoacoustic waves into electric signals.

Moreover, in this embodiment, these acoustic elements also have the function of transmitting the ultrasound waves to the object 101. In addition, ultrasound signals can be obtained by these acoustic elements detecting the ultrasound echoes that were reflected inside the object and converting such reflected ultrasound echoes into electric signals. The foregoing photoacoustic wave signal corresponds to the first electric signal of the present invention and the foregoing ultrasound signal correspond to the second electric signal of the present invention.

In the present invention, the probe 102 may be configured from any kind of system. For example, a conversion element utilizing piezoelectric ceramics (PZT), which are used in a standard ultrasound diagnostic device, can be used. Moreover, a capacitance-type capacitive micromachined ultrasound transducer (CMUT) may also be used. Moreover, an magnetic MUT (MMUT) which uses a magnetic film or a piezoelectric MUT (PMUT) which uses a piezoelectric thin film may also be used.

Note that the probe 102 is preferably a probe that is configured so that it can transmit ultrasound waves and detect both ultrasound echoes and photoacoustic waves, and it is thereby possible to obtain object information of ultrasound waves and photoacoustic waves of the same position. However, the target of application of the present invention is not limited to the above. Signal processing may also be performed by installing separate probes for sending and receiving ultrasound waves and receiving photoacoustic waves, respectively.

Moreover, it is preferable to use an array-type probe in which a plurality of acoustic elements are arranged two-dimensionally so that the photoacoustic waves that are generated and propagate three-dimensionally from a generation source such as the light absorbing material can be detected at the broadest possible solid angle. As a result of using the array-type probe, it is possible to acquire the volume data of the photoacoustic waves and the ultrasound waves that is required for imaging the object region of the front face of the probe. However, a linear scan-type probe in which the acoustic elements are arranged linearly may also be used.

Since the space between the probe 102 and the object 101 is the propagation path of the photoacoustic waves and the ultrasound waves, it is preferable to dispose an acoustic matching material to obtain a strong acoustic bond. As the acoustic matching material, for instance, gel or a gel sheet for measuring ultrasound waves may be used.

(Optical system)

The light source 103 emits pulsed light (width 100 nsec or less) having a center wavelength in a near infrared region of 530 to 1300 nm. As the light source 103, generally used is a solid-state laser (for example, Yttrium-Aluminum-Garnet laser or Titan-Sapphire laser) capable of pulse emission having a center wavelength in the near infrared region. Lasers such as gas laser, pigment laser, and semiconductor laser may also be used, or a light-emitting diode or the like may be used as the light source 103 in substitute for a laser.

Note that the optical wavelength is selected between 530 and 1300 nm according to the light absorbing material in the living body to be measured. The light absorbing material may be, for example, oxidized hemoglobin or deoxygenated hemoglobin or blood vessels that contain a large amount of the same, a malignant tumor containing many neovascular vessels, or otherwise glucose, cholesterol and the like. For example, upon measuring the hemoglobin in the breast cancer neovascular vessels, light of 600 to 1000 nm is generally absorbed, and, on the other hand, since the light absorption of water configuring the living body will become extremely small near 830 nm, light absorption will relatively increase at 750 to 850 nm. Moreover, since the light absorption rate will change for each optical wavelength depending on the state of the hemoglobin (oxygen saturation), functional changes of the living body can also be measured by using the foregoing wavelength dependency.

The irradiation optical system 104 guides the pulsed light emitted from the light source 103 toward the object, and forms and irradiates light 121 that is suitable for measurement. The irradiation optical system 104 is typically configured from optical components such as a lens or a prism which converges or expands light, a mirror which reflects light, or a diffuser which diffuses light. Moreover, as the light guide from the light source 103 to the object 101, it is also possible to use a light waveguide such as an optical fibre.

Note that, as a safety standard related to the irradiation of laser beams and the like with which skin or eyes are irradiated, the maximum permissible exposure is set forth in IEC60825-1 generally based on conditions such as the optical wavelength or exposure duration, pulse repetition and the like. The irradiation optical system 104 generates the light 121, upon ensuring safety in accordance with the foregoing standard, of a shape and output angle which are suitable for imaging the object region of the front face of the probe 102. In FIG. 1, while the irradiation optical system 104 is disposed only on one side of the probe 102, in reality the disposition thereof is not limited thereto. For example, by causing either side of the probe 102 to be subject to photo irradiation, the object region of the front face of the probe 102 can be illuminated more uniformly.

Moreover, the irradiation optical system 104 comprises an optical configuration not shown which detects the emission of the light 121 to the object 101, and generates a synchronization signal for synchronously controlling the receiving and recording of the photoacoustic wave signals. The emission of the light 121 can be detected by splitting a part of the pulsed light generated by the light source 103 using an optical system such as a half mirror and guiding the split pulsed light to the optical sensor, and using the detection signals generated by the optical sensor. When using a bundle filter for guiding the pulsed light, a part of the fiber can be branched and guided to the optical sensor for detecting the emission of the light 121. The synchronization signals generated by the foregoing detection are input to the signal reception unit 105.

(Signal processing system)

The signal reception unit 105 amplifies the photoacoustic wave signals or the ultrasound signals generated by the probe 102 according to the synchronization signals sent from the irradiation optical system 104 or the ultrasound transmission controller 107, and converts the amplified signals into digital signals. The signal reception unit 105 is configured from a signal amplifies which amplifies the analog signals generated by the probe 102, and an A/D converter which converts analog signals into digital signals.

The photoacoustic wave signal processor 106 performs, on the digital signals of the photoacoustic waves generated by the signal reception unit 105, correction of variation in sensitivity of the acoustic elements of the probe 102, complementary processing of elements that are physically or electrically defective, integration processing for reducing noise, and so on. By repeating the acquisition of photoacoustic wave signals at the same position of the object 101 based on integration processing and subsequently performing integration average processing, it is possible to reduce the system noise and improve the S/N ratio of the photoacoustic wave signals.

The photoacoustic signals that are obtained by detecting the photoacoustic waves generated from the light absorbing material inside the object 101 are generally weak signals. Thus, by irradiating the light 121 a plurality of times and integrating the plurality of photoacoustic wave signals that were obtained in the respective irradiations to improve the S/N ratio, it is possible to obtain a photoacoustic wave image that is suitable for image diagnosis.

The ultrasound transmission controller 107 generates drive signals to be applied to the individual acoustic elements configuring the probe 102, applies the generated drive signals to the acoustic elements, and controls the frequency of the ultrasound waves to be transmitted and the sound pressure. In this embodiment, in an array-type probe in which a plurality of acoustic elements are arranged two-dimensionally, a linear scan of the transmission of ultrasound beams and reception of ultrasound echoes along one direction configuring the arrays is performed, and such linear scan is performed once again in the other direction. It is thereby possible to obtain a three-dimensional ultrasound wave data that is configured from a plurality of B-mode images.

The ultrasound transmission controller 107 comprises a transmission control function of setting the transmission direction of the ultrasound beams and setting the transmission delay pattern in correspondence with the transmission direction, and a reception control function of setting the reception direction of the ultrasound echoes and selecting the reception delay pattern in correspondence with the reception direction. The transmission delay pattern is a pattern of the delay time that is given to a plurality of drive signals for forming the ultrasound beams in a predetermined direction based on the ultrasound waves that are transmitted from the plurality of acoustic elements. Moreover, the reception delay pattern is a pattern of the delay time that is given to a plurality of reception signals for extracting the ultrasound echoes from an arbitrary direction relative to the ultrasound signals that are detected by the plurality of acoustic elements. The transmission delay pattern and the reception delay pattern are stored in the storage unit 113.

The ultrasound signal processor 108 performs receiving focus processing of associating the respective delay times with the digital signals of the ultrasound waves generated by the signal reception unit 105 based on the reception delay pattern selected by the ultrasound transmission controller 107, and adding the respective signals. Based on this processing, focused ultrasound wave data can be generated. In addition, the B-mode images are generated through logarithmic compression, filter processing, and so on.

The control processor 109 runs an operating system (OS) for controlling and managing the basic resources in the program operation. In addition, the control processor 109 reads the program codes stored in the storage unit 113, and executes the function of the embodiments described later. In particular, the control processor 109 manages the acquisition operation of object information upon receiving an event notice that is issued based on various operations such as commands for starting or discontinuing imaging that are input by the user via the operation unit 110, and controls the various types of hardware via the system bus 114.

Moreover, the control processor 109 acquires parameters for the acquisition of object information which are designated by the operation unit 110, or set in advance in the storage unit 113. In addition, the control processor 109 outputs, to the light source 103 and the photoacoustic wave signal processor 106, laser emission control information which is required for satisfying the integration count of the photoacoustic wave signals based on the foregoing parameters. Similarly, the control processor 109 outputs, to the ultrasound transmission controller 107 and the ultrasound signal processor 108, control information related to the ultrasound wave transmission/reception control operation such as a plurality of focus settings in the transmission of ultrasound beams and the reception of ultrasound echoes.

Otherwise, the controller processor 109 manages identifying information for identifying the individual apparatuses and information that is set uniquely for each apparatus, and monitors the condition of the individual hardware in order to manage the apparatus condition.

The image component 111 configures an arbitrary tomographic image of the photoacoustic wave image and the ultrasound image by imaging the tissue information in the object based on the digital signals and ultrasound wave data of the acquired photoacoustic waves, or a superimposed display image thereof. Moreover, the image component 111 configures information that is more suitable for the diagnosis by applying various types of correction processing to the configured image such as brightness correction, distortion correction, cutout of the focus region and the like. Moreover, the image component 111 adjusts the photoacoustic wave image or the ultrasound image, or the parameters or display image related to the configuration of the superimposed image thereof in accordance with the operations of the operation unit 110 performed by the user. The photoacoustic wave image corresponds to the first image of the present invention, and the ultrasound image corresponds to the second image of the present invention.

The photoacoustic wave image can be obtained by performing image reconstruction processing on the digital signals of three-dimensional photoacoustic waves that were generated as a result of being detected by the plurality of acoustic elements arranged in an array, respectively. The photoacoustic wave image can visualize object information of the characteristic distribution such as the acoustic impedance or optical characteristic value distribution. As the image reconstruction processing, for instance, back projection based on time domain or Fourier domain which is generally used in the tomography technology, or phasing addition processing may be used. Note that, if there is no strict time limit, an image reconstruction method such as the inverse problem analysis method performed via repeated processing may also be used, and the object information can also be visualized without performing image reconstruction as a result of using a probe comprising a receiving focus function using an acoustic lens or the like.

The image component 111 is generally configured using a graphics processing unit (GPU) or the like having a sophisticated arithmetic processing function and graphic display function. It is thereby possible to shorten the time required to perform the image reconstruction processing or configure the display image.

The storage unit 113 is configured from a memory that is required for the control processor 109 to operate, a memory for temporarily retaining data during the object acquisition operation, and a hard disk for storing and retaining the generated photoacoustic wave image and ultrasound image, related object information and diagnosis information, and so on. In addition, the storage unit 113 stores the program codes of the software which realizes the functions of the embodiments described later. Moreover, the storage unit 113 stores factory default values and default parameters as the parameters related to the acquisition operation of object information. The default parameter reflects the parameter settings that are often used when the acquisition operation of object information is repeatedly performed, and may be suitably updated by the user. As a result of retaining the default parameter, it is possible to avoid the trouble of setting all parameters each time object information is acquired.

Note that the default value may also be stored and retained for each object information acquiring apparatus, or for each piece of connected hardware such as the probe 102, or for each object 101 or user.

(Interface)

The display unit 112 displays the photoacoustic wave image and the ultrasound image constructed by the image component 111 or the superimposed image thereof, and an UI for operating such images and the apparatus. While a liquid crystal display is generally used, an organic electro luminescence (EL) display and other display systems may also be used.

The operation unit 110 is an input device for the user to designate the integration count of the photoacoustic wave image or the parameters related to the acquisition operation of object information such as the respective reception gain settings of photoacoustic waves and ultrasound waves. Moreover, the operation unit 110 comprises a corrector 110A for photoacoustic waves and a corrector 110B for ultrasound waves, and also comprises a function for image processing operation related to the images. The operation unit 110 is generally configured from a mouse, a keyboard, a touch panel and the like, and sends, according to the user's operation, an event notice to the software such as the OS or the like that is running on the control processor 109.

In the object information acquiring apparatus configured as described above, by using a probe that can detect photoacoustic waves and ultrasound waves, the photoacoustic wave image and the ultrasound image of the same object region can be collectively acquired in a single acquisition operation of object information, excluding cases where the position or posture of the probe is changed. Moreover, by displaying the same photoacoustic wave image and ultrasound image side by side or generating and displaying the superimposed image thereof, it is possible to support the user's observation or analysis or diagnosis of the object information. In addition, by providing independent brightness correctors to for the photoacoustic wave image and the ultrasound image, brightness correction which enables easier observation of images by the user can be realized without having to depend on the attenuation characteristics based on the respective measurement principles.

(Processing flow)

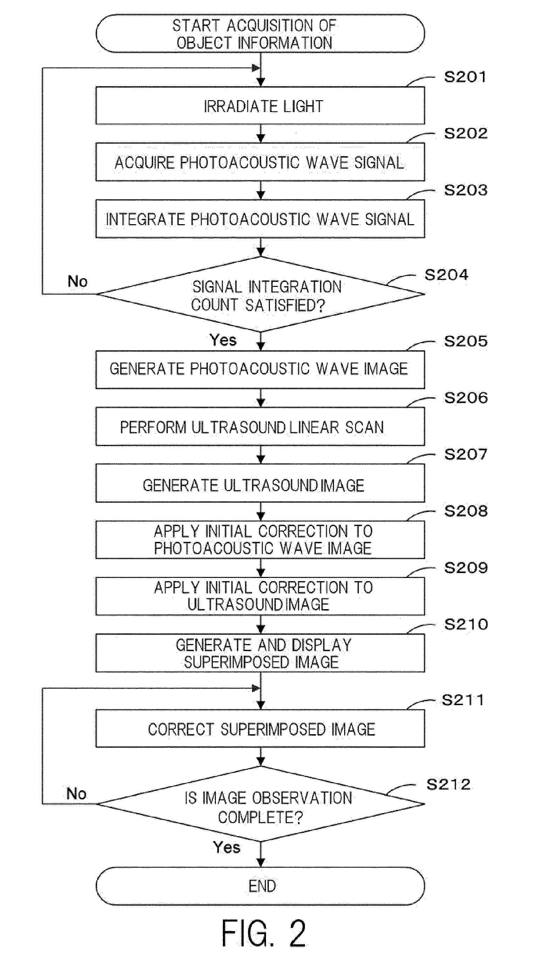

The flow of acquiring object information in Embodiment 1 is now explained with reference to FIG. 2. The flowchart of FIG. 2 is performed when the user commands the start of acquisition of object information via the operation unit 110.

In step S201, the light source 103 irradiates pulsed light according to the emission start command from the control processor 109 pertaining to the acquisition of object information. The pulsed light emitted from the light source 103 is shaped by the irradiation optical system 104 and irradiated as light 121 with which the object 101 is irradiated. The irradiation optical system 104 generates synchronization signals simultaneously with the emission of the light 121 to the object 101, and sends the generated synchronization signals to the signal reception unit 105.

In step S202, the photoacoustic wave signal is acquired. Specifically, the probe 102 foremost detects the photoacoustic waves generated from the object 101, and generates analog electric signals (photoacoustic wave signals). Subsequently, the signal reception unit 105 starts receiving the photoacoustic wave signals in synch with the synchronization signals generated by the irradiation optical system 104, and converts the received photoacoustic wave signals into digital signals. Note that the signal reception unit 105 that received the synchronization signals receives photoacoustic wave signals in an amount of a predetermined number of samples at a sampling rate that can be operated from that moment. The number of samples is determined in light of the propagation rate of the acoustic waves in the object and the maximum measured depth as the apparatus specification.

In step S203, the photoacoustic wave signal processor 106 performs integration processing by using the digital signals of the photoacoustic waves that were generated in step S202.

In step S204, whether the signal integration count of the photoacoustic waves that was designated by the user or set in advance has been satisfied is determined. When the signal integration count has not been satisfied, the processing proceeds to step S201 and acquisition of the photoacoustic wave signals is repeated. When the signal integration count has been satisfied, the processing proceeds to step S205.

In step S205, the image component 111 generates a photoacoustic wave image by using the integrated photoacoustic wave signals that are obtained by step S204. Note that, generally speaking, reconstruction processing requires much time, and, since the processing can be entrusted to the GPU, it is also possible to perform the subsequent processing in parallel. Upon generating an image, for instance, the universal back-projection (UBP), which is an image reconstruction method using a time domain, may be used.

In step S206, the object is scanned with ultrasound waves. Specifically, the control processor 109 foremost commands the ultrasound transmission controller 107 to start the acquisition of the ultrasound wave B-mode image. Subsequently, the ultrasound transmission controller 107 performs a linear scan of the transmission of ultrasound beams and the reception of ultrasound echoes based on parameters such as a plurality of focus settings. Moreover, the ultrasound transmission controller 107 sends the synchronization signals to the signal reception unit 105 simultaneously with the transmission of the ultrasound beams. The ultrasound echoes are detected by the probe 102, and the signal reception unit 105 that received the synchronization signals receives the ultrasound signals in an amount of a predetermined number of samples at an operable sampling rate, and converts the received ultrasound signals into digital signals.

In step S207, the ultrasound signal processor 108 generates the B-mode image (ultrasound image) by performing phasing addition processing, logarithmic compression, filter processing and the like on the digital signals of the ultrasound waves that were obtained in step S206.

In step S208, initial correction is performed on the photoacoustic wave image. The initial correction is correction processing that is performed for each ultrasound image and photoacoustic wave image in accordance with their characteristics. As an example of the initial correction of the photoacoustic wave image, there is processing of applying gain to the intensity of the photoacoustic wave that were detected from the segment to be reconstructed in accordance with the amount of light that reaches the segment. Upon performing the initial correction, the default correction value that is retained in the storage unit 113 in advance, or the correction value that is calculated upon estimating the optical attenuation characteristics is applied to the photoacoustic wave image. The initial correction may be performed with a constituent element having an image processing function such as the photoacoustic wave signal processor 106 or the image component 111.

Note that the optical attenuation characteristics in the living body (distribution of the optical attenuation coefficient); that is, the scattered shape of the light 121 is calculated logically based on the transport equation, or based on the optical diffusion equation that is derived based on approximation. The optical attenuation coefficient may be calculated using the finite element method, which is a numerical calculation method, based on information such as the optical wavelength of the light 121 or the shape of light upon entering the object. Since the shape of the light 121 upon entering the object is determined based on the design of the irradiation optical system 104, the information may also be the design data of the irradiation optical system 104. Otherwise, the information may also be data that is measured using an energy meter or the like capable of measuring the light shape. The shape data is stored in the storage unit 113 in advance.

In step S209, the initial correction is performed on the ultrasound image. Here, the default correction value that is retained in the storage unit 113 in advance, or the correction value that is calculated in consideration of the signal attenuation of the ultrasound waves indicating the characteristics of the basic exponential function is applied to the ultrasound image. Note that the correction value calculated in steps S208 and S209 is reflected, together with the image display, at the initial position of the correction value control points 304A to 304G and 308A to 308G on the UI described later.

In step S210, the image component 111 generates a superimposed image by using the photoacoustic wave image and the ultrasound image which have been corrected in steps S208 and S209. The generated superimposed image is displayed on the display unit 112.

In step S211, the correction processing of the superimposed image is performed. The details thereof will be explained later.

In step S212, whether an event of operation for ending the image observation for the diagnosis of breast cancer has been notified is determined. When the image observation is complete, the series of processing for acquiring the object information is ended. When image observation is not complete and is to be continued, the processing proceeds to step S211 to repeat image correction, and the image display is continued.

Based on the foregoing processing, two types of object information based on the photoacoustic wave image and the ultrasound image can be collectively acquired in a single acquisition operation of object information.

Note that, in this flowchart, while the acquisition of the ultrasound image was started after the photoacoustic wave image has been generated, there may be cases where the ultrasound image can be acquired during the course of repeating the reception of the photoacoustic wave signals for integration. The reception cycle of the photoacoustic wave signals is determined based on the repetition frequency of the emission of the light source 103 and, for example, the cycle will be 100 msec with a laser capable of pulse emission at 10 Hz. For example, when the reception time of the photoacoustic wave signals is 30 $\mu$sec, there will be a standby time of roughly 99 msec until the reception of the subsequent photoacoustic wave signal. When the time required for the transmission of ultrasound beams and the reception of ultrasound echoes is, for example, 60 $\mu$sec, when the transmission/reception of ultrasound waves is performed 128 times for generating the B-mode image, the reception of the ultrasound signals can be completed in roughly 100 $\mu$sec $\times$ 128 lines = 12.8 msec. Thus, the photoacoustic wave signals and the ultrasound signals can be performed based on a time division in a cycle of 100 msec.

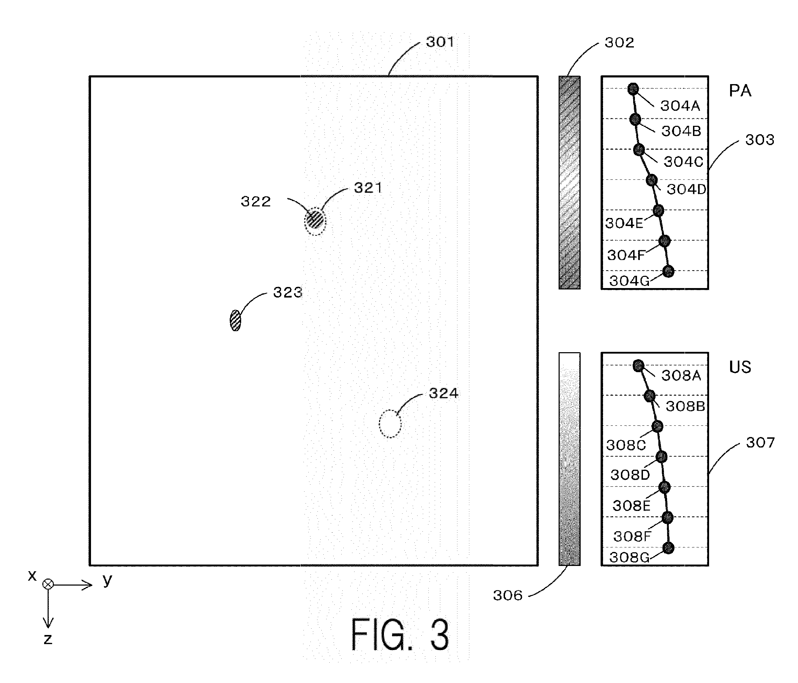

FIG. 3 is a conceptual diagram explaining the corrector of object information in Embodiment 1. The corrector in this embodiment is provided as a UI that is displayed on the display unit 112. Note that the application of this invention is not limited to the configuration based on the UI of software, and the corrector function may also be provided with hardware such as a slide bar or a dial.

Reference numeral 301 shows a superimposed image of the photoacoustic wave image and the ultrasound image of an arbitrary cross section of the object. For example, among the B-mode images of the ultrasound waves acquired by the probe 102, one center B-mode image and a tomographic image of the photoacoustic wave image showing the same object region are superimposed to obtain the superimposed image. The superimposed image 301 in this embodiment is, for example, an image which visualizes an object region having a width of 32 mm (y axis direction) $\times$ depth of 40 mm (z axis direction). Moreover, let it be assumed that the photoacoustic wave image and the ultrasound image are constructed based on a 12-bit gradation/4096 gradation per pixel. Note that, in FIG. 3, the z axis shows the depth direction; that is, the measured depth direction viewed from the probe surface. Accordingly, the value of the z axis shows the distance from the probe. Moreover, the y axis shows the same direction as the linear scan direction of the ultrasound wave.

The superimposed image is an image that is obtained by superimposing the photoacoustic wave image over the ultrasound image as the base. The ultrasound image shows highly precise form information related to the profile shape of the tissues in the object and the position in the depth direction (distance from the probe), and so on. The photoacoustic wave image visualizes the distribution of active angiogenesis caused by breast cancer and shows the function size of the amount of oxygen in the blood, and so on. Thus, in the diagnosis using a superimposed image, it is possible to intuitively associate the form of tissues and the function size thereof and observe the same.

Note that, upon superimposing the photoacoustic wave image over the ultrasound image, the comprehension of the image during the diagnosis can be facilitated by setting the opaque lightness of the photoacoustic wave image. Here, a method of determining the opaque lightness of the pixels in a manner of remaining in correspondence with the brightness value of the photoacoustic wave image, or a method of causing the brightness value region, which has a given value or less and does not indicate a significant function size, to become transparent, may be adopted.

In the superimposed image, generally speaking, the brightness value of the ultrasound image indicating the information on the form of tissues is indicated with a gray scale that directly shows light/shade, and the brightness value of the photoacoustic wave image indicating the function size is indicated, for example, in a color shade from blue as the minimum value to green as the intermediate value and to red as the maximum value. It is thereby possible to intuitively comprehend the image.

In the superimposed image 301 of FIG. 3, shown are light absorbing materials (reference numerals 322, 323) visualized in the photoacoustic wave image, and tissues (reference numerals 321, 324) visualized in the ultrasound image.

Reference numeral 302 is the color scale as a legend of the brightness value of the photoacoustic wave image. Reference numeral 303 shows the corrector of the photoacoustic wave image, and is a UI corresponding to the photoacoustic wave corrector 110A. Moreover, reference numeral 306 is the gray scale as the legend of the brightness value of the ultrasound image. In addition, reference numeral 307 shows the corrector of the ultrasound image, and is a UI corresponding to the ultrasound wave corrector 110B. The correctors 303 and 307 in Embodiment 1 are respectively correctors for correcting the brightness values of the photoacoustic wave image and the ultrasound image.

Within the corrector 303 of the photoacoustic wave image, control points for setting the correction values of 304A, 304B, 304C, 304D, 304E, 304F, 304G are disposed. The user can individually set the correction values according to the depth relative to the photoacoustic wave image. By operating the control points 304A to 304G, it is possible to turn off the display of the light absorbing material 322 or 323, or highlight the same.

Note that, in relation to the attenuation characteristics relative to the depth direction of the photoacoustic wave image, the attenuation characteristics of light and the attenuation characteristics of the photoacoustic waves respectively show the exponential characteristics. Thus, the correction value curve (arrangement of the control points 304A to 304G) relative to the foregoing combination will basically be an arrangement along a logarithmic function.

Moreover, similarly within the corrector 307 of the ultrasound image, control points for setting the correction values of 308A, 308B, 308C, 308D, 308E, 308F, 308G are disposed. The user can individually set the correction values according to the depth relative to the ultrasound image. By operating the control points 308A to 308G, it is possible to turn off the display of the tissue 321 or 324, or highlight the same.

Note that, in relation to the attenuation characteristics relative to the depth direction of the ultrasound image, the attenuation of the ultrasound waves shows the exponential characteristics. Thus, the correction value curve (arrangement of the control points 308A to 308G) will basically be a logarithmic arrangement.

When the user observes the superimposed image 301, there are cases where the user may wish to highlight an image with a high brightness, or weakly display the same with a low brightness for comparison. Thus, the corrector 303 of the photoacoustic wave image and the corrector 307 of the ultrasound image are preferably disposed close to each other. In FIG. 3, while the corrector 303 of the photoacoustic wave image and the corrector 307 of the ultrasound image are disposed on top of each other vertically, the correctors 303 and 307 can alternately operated with easy by being disposed side by side, and the complicated user operation during the image observation can be eliminated.

Moreover, when the correctors 303 and 307 are operated while observing the superimposed image 301, it would be undesirable for the line of sight to move considerably and the superimposed image 301 to deviate from the visual field. Thus, as shown in FIG. 3, by disposing the superimposed image 301 and the correctors 303 and 307 side by side, it is possible to view the correctors 303 and 307 with a peripheral vision in the visual field of observing the superimposed image 301, or reduce the movement of the line of sight as much as possible.

Note that, in FIG. 3, while seven control points were disposed relative to the depth direction of the image in the correctors 303 and 307 to provide the correction value setting function, such control points may be disposed in a required quantity according to the depth to be measured or the like. Moreover, a difference number of control points may be disposed in the correctors 303 and 307.

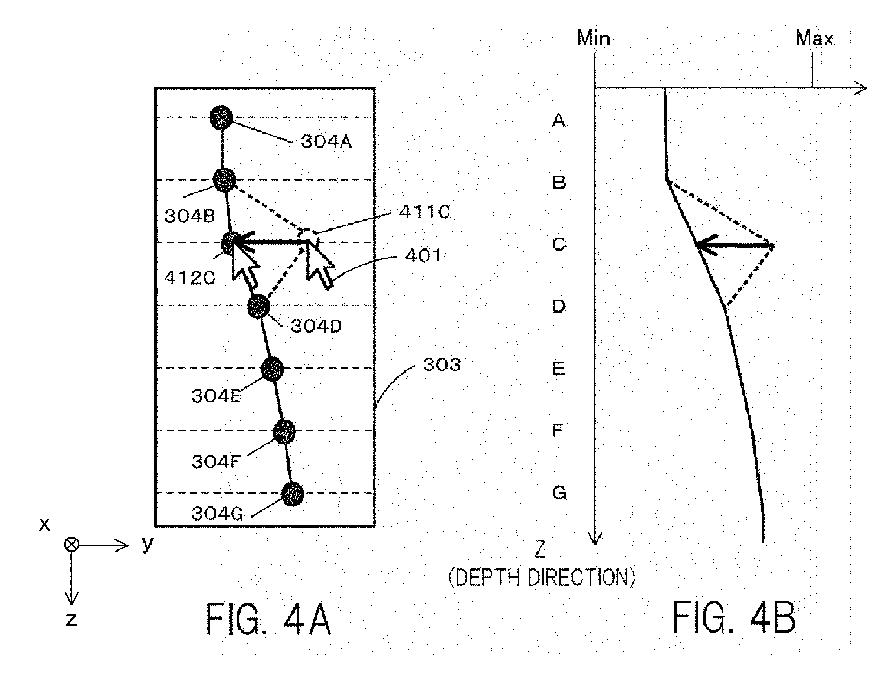

The correction method of objection information in this embodiment is now explained with reference to FIG. 4. FIG. 4A shows a case of operating the corrector 303 of the photoacoustic wave image shown in FIG. 3, and FIG. 4B shows a case of the correction value curve that is calculated as a result of that operation, respectively.

The user sets the correction values by drag-operating the control points 304A, 304B, 411C, 304D, 304E, 304F, 304G via the operation unit 110. When the user drag-operates the respective control points, the corrector 303 notifies a value change event of positions to the control processor 109. For example, while the user is drag-operating the control point 411C, the corrector 303 successively notifies the change in the value which indicates the position of the control points. The control processor 109 that received the foregoing notice successively generates and displays a photoacoustic wave image reflecting the new correction value after the foregoing operation.

In this embodiment, the effect of brightness correction increases when the control points are moved rightward, and the effect of the brightness correction weakens when the control points are moved leftward. For example, when the control point 411C is moved up to the setting fixed position 412C, the correction value in the depth position C will decrease.

After the values of the seven control points disposed discretely along the depth direction of the photoacoustic wave image are set, when the correction values among the control points are subject to linear interpolation, the correction value curve of FIG. 4B is calculated. In the example of FIG. 4B, nearest neighbor interpolation is applied on a region that is shallower than the control point 304A and on a region that is deeper than the control point 304G. Note that the interpolation of discrete control points may be performed using well-known interpolation methods such as nearest neighbor interpolation, secondary interpolation, tertiary interpolation, or polynomial interpolation.

In FIG. 4B, the vertical axis shows the depth position of the photoacoustic wave image, and the horizontal axis shows the correction value, respectively. The brightness value can be used as the correction value. For example, the Min value of the correction value (brightness value) may be set as 0 and the Max value may be set as 4095, and the individual pixel brightness values configuring the photoacoustic wave image may be simply added for performing the correction.

Magnification may also be used as the correction value. For example, the Min value of the correction value (magnification) may be set to 0.2 and the Max value may be set to 1.8, and the intermediate value thereof may be set to 1.0, and the individual pixel brightness values may be multiplied for performing the correction.

In the present invention, since the user can control the correction value to be an intended value, adjustment can be made so that the image is easy to view for that user. Note that, when the upper limit of gradation of the color scale 202 is exceed by reflecting the correction value, the upper limit value of the display gradation may be displayed.

The user can perform brightness correction on the ultrasound image by performing similar operations in the corrector 307 of the ultrasound wave.

In addition, by independently comprising the corrector 303 for photoacoustic waves and the corrector 307 for ultrasound waves, it is possible to obtain a photoacoustic wave image and an ultrasound image having a uniform brightness level regardless of the depth, and this contributes to the improvement in the visibility of the superimposed image.

Note that, in FIG. 4, while a case where the user sets the correction value by operating the operation unit 110 by depending on the pointer 401 was explained, the user may also directly perform the operations with one's finger by using a touch panel. Moreover, the user can also operate the correction values by using a keyboard and moving the focus to the control points using the TAB key or the like, and thereafter pressing down the cursor key.

As described above, the brightness correction of the photoacoustic wave image and the brightness correction of the ultrasound image can be performed individually busing the operation unit 110 according to the UI on the display unit 112. Moreover, since the image is only subject to brightness correction, it is possible to swiftly respond to the user's operation of the correction value.

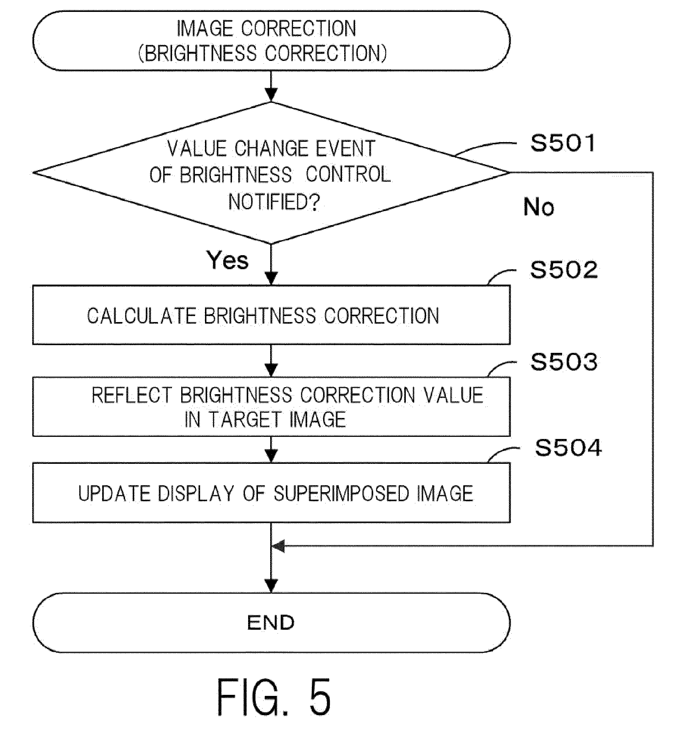

FIG. 5 is a flowchart showing the flow for correcting the object information; that is, the image correction (brightness correction) flow in this embodiment.

In step S501, the control processor 109 determines whether a value change event of the brightness correction control points 304A to 304G of the photoacoustic wave image or the brightness correction control points 308A to 308G of the ultrasound image was notified. When a value change event was notified, the processing proceeds to step S502. When a value change event was not notified, the processing is ended.

In step S502, the control processor 109 calculates the brightness correction value shown in FIG. 4B according to the value of the brightness correction control points notified in step S501. Note that, when the control points of the photoacoustic wave image were operated, the calculation of the brightness correction value is omitted since it will be sufficient to maintain the current setting value with regard to the ultrasound image. The same applies vice versa.

In step S503, the brightness correction value calculated in step S502 is reflected by the control processor 109 in the respective brightness values. The brightness value of the photoacoustic wave image is corrected when an event occurs in the control points 304A to 304G, and the brightness value of the ultrasound image is corrected when an event occurs in the control points 308A to 308G.

In step S504, the display of the superimposed image on the display unit 112 is updated in related to the target image that was updated based on the new brightness correction in step S503.

In the object information acquiring apparatus configured as described above, by providing independent brightness correctors to for the photoacoustic wave image and the ultrasound image, brightness correction which enables easier observation of images by the user can be realized. Moreover, automatic correction according to the respective attenuation characteristics of the photoacoustic waves and the ultrasound waves can be applied as the initial correction upon presenting images to the user during a single acquisition of object information.

As explained with reference to each of the foregoing flowcharts, the photoacoustic wave image is subject to the initial correction by the photoacoustic wave signal processor or the image component or corrected based on the user's operation through the operation unit (operation unit) or the photoacoustic wave corrector. In the present invention, these constituent elements correspond to the first corrector. Similarly, the ultrasound signal processor, the image component, the ultrasound wave corrector of the operation unit and so on correspond to the second corrector of the present invention.

In this embodiment, since the correction value responds in real-time by following the operation of the control points, the user can intuitively correct the brightness of the photoacoustic wave image and the ultrasound image. In addition, upon observing the superimposed image, by the user changing the brightness correction of one image in a short period of time, it is possible to assist the user to visually confirm the difference in comparison with the other image and the superimposition of the functional information and the form information of the tissues in the object.

Note that, in this embodiment, while a case of presenting the photoacoustic wave image and the ultrasound image as a superimposed image to the user was explained, the application of the present invention is not limited thereto. The photoacoustic wave image and the ultrasound image may also be presented side by side, or the images may be otherwise presented so that the user can compare the two images.

Moreover, in this embodiment, while a mode of correcting the brightness of both the photoacoustic wave image and the ultrasound image according to the distance from the probe was explained, the application of the present invention is not limited thereto. The correction may be performed based on any kind of index so as long as it is possible to correct the influence on the brightness of both the photoacoustic wave image and the ultrasound image. For example, when correcting the influence on the brightness caused by optical attenuation, the brightness of the photoacoustic wave image may be corrected according to the distance from the surface of the object, which is the region that is irradiated with light.

<Embodiment 2>

Embodiment 2 of the present invention is now explained with reference to the drawings.

In Embodiment 1 described above, the attenuation characteristics based on the respective measurement principles were estimated in the acquisition processing of the photoacoustic wave image and the ultrasound image, and the depth correction according to the estimated attenuation characteristics was thereafter automatically applied as the initial correction and presented to the user.

In this embodiment, for instance, when displaying previously stored photoacoustic wave image data, the initial correction is performed based on the image characteristics such as the brightness distribution thereof. The characteristic portions of the present invention are now mainly explained.

Note that the apparatus configuration of the object information acquiring apparatus in this embodiment is the same as the apparatus configuration shown in FIG. 1, and the explanation thereof is omitted. Moreover, the UI to be presented to the user can also be implemented in the modes explained with reference to FIG. 3 and FIG. 4.

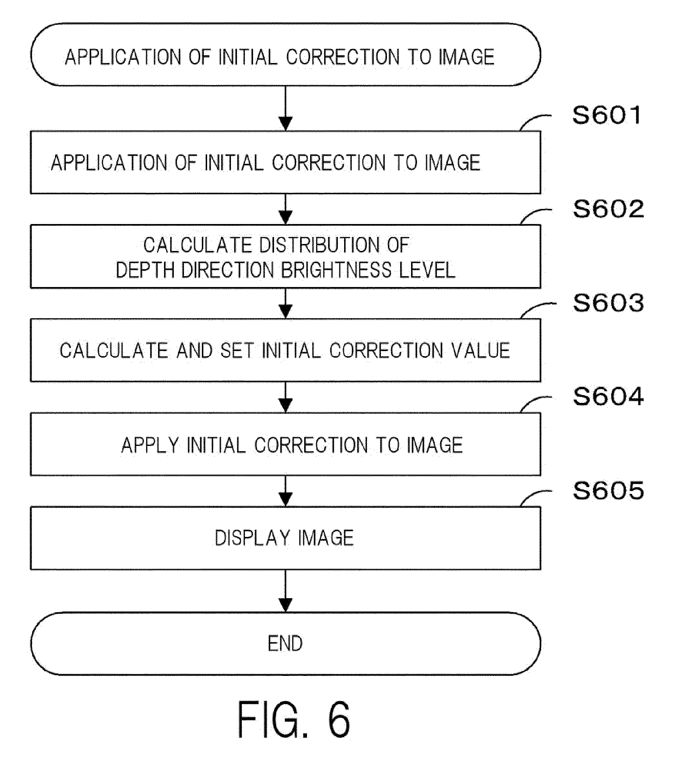

FIG. 6 is a flowchart explaining the method of applying initial correction in the object information, particularly in the photoacoustic waves, in this embodiment.

In step S601, the control processor 109 reads the signal data that is required for generating the photoacoustic wave image stored in the storage unit 113. Note that the photoacoustic wave image data stored in the storage unit 113 is not the image data that was subject to the brightness correction according to the method of Embodiment 1, and is pre-corrected data that is generated from the signal data of the received photoacoustic waves.

In step S602, the control processor 109 calculates the average value of a plurality of pixels in the same depth position (position in the z direction shown in FIG. 3) of the image data read in step S601. Otherwise, the control processor 109 calculates the brightness level for each region upon evenly dividing the pixels into seven regions relative to the depth direction. The average value thereof basically represents the brightness level of that region. The brightness distribution can thereby be obtained. Here, the control processor corresponds to the brightness distribution calculation unit of the present invention. The brightness distribution calculation unit obtains the first brightness distribution of the present invention from the photoacoustic wave image, and obtains the second brightness distribution of the present invention from the ultrasound image.

In step S603, the control processor 109 calculates the correction value so that the brightness level becomes uniform regardless of the depth direction based on the brightness level of each of the calculated depth positions. To put it differently, obtained is a correction value according to the brightness distribution according to the distance from the probe. For example, when the brightness level is to be unified with an intermediate value as the reference in the brightness levels calculated for each depth position, as the correction value of the brightness level exceeding the intermediate value, a negative correction value in the amount of such difference is assigned, and, contrarily, a positive correction value in the amount of such difference is assigned to the brightness level falling below the intermediate value. Moreover, for example, the correction may also be performed so as to unify the brightness levels of a relatively outer surface region of the object 101, which can retain a high S/N in the reception of the photoacoustic waves, as the reference. The reference in the photoacoustic wave image corresponds to the first reference of the present invention. Similarly, the reference of the ultrasound image corresponds to the second reference of the present invention.

In step S604, as the initial correction of the photoacoustic wave image, the correction value calculated by the control processor 109 in step S603 is applied to the photoacoustic wave image data that was read in step S601.

In step S605, the image component 111 displays, on the display unit 112, the photoacoustic wave image that was corrected in step S604. Note that it is also possible to display a superimposed image with ultrasound images that were collectively acquired in the acquisition of the same object information in the past. Moreover, the correction value calculated in step S603 is reflected, together with the image display, in the initial position of the correction value control points 304A to 304G and 308A to 308G on the UI described later.

According to the correction method of object information configured as described above, it is possible to calculate the initial correction value based on changes in the brightness in the depth direction upon reading and displaying a previously stored photoacoustic wave image, and apply the initial correction value to the photoacoustic wave image. In an object, the amount of light and the attenuation characteristics of photoacoustic waves and ultrasound waves are hardly ever uniform. Nevertheless, according to this embodiment, it is possible to prevent, to the user, a photoacoustic wave image having a uniform brightness level across the entire measured depth without having to estimate the foregoing characteristics. Note that, in this embodiment, while the brightness level was represented using the average brightness value, the brightness value with the greatest amount of distribution based on a histogram may also be used as the brightness level.

In addition, the initial correction can be similarly applied to the ultrasound image.

<Embodiment 3>

Embodiment 3 of the present invention is now explained with reference to the drawings. The correction of object information in this embodiment reflects the correction value of the corrector in the received gain control of the signals; that is, in the TGC of the signal processor. The characteristic portions of the present invention are now mainly explained.

Note that the apparatus configuration of the object information acquiring apparatus in this embodiment is the same as the apparatus configuration shown in FIG. 1, and the explanation thereof is omitted. Moreover, the UI to be presented to the user can also be implemented in the modes explained with reference to FIG. 3 and FIG. 4.

Note that the signal processor in this embodiment includes, in addition to the function of the foregoing embodiments, a function of being able to perform time gain control (TGC) of increasing or decreasing the amplification gain in accordance with the time required for the acoustic waves to reach the probe 102. Generally speaking, a variable gain amplifier for controlling the amplification gain based on voltage control is used as the circuit for performing TGC. As a result of configuring the amplifier of the detection signals of the acoustic waves from a variable gain amplifier, it is possible to perform TGC for controlling the signals for use in variable gain control simultaneously with the reception time of the acoustic waves.

TGC is a technique that is generally used in an ultrasound diagnostic device, and its objective is to correct the attenuation of the ultrasound waves in the object. Specifically, monotonic increase gain control is performed on the exponential attenuation characteristics of the ultrasound waves.

Note that, according to the technology of Japanese Patent Application Laid-Open No. 2010-015535, it is also possible to perform TGC of increasing or decreasing the amplification gain of the photoacoustic waves according to the time required for such photoacoustic waves to reach the probe upon giving consideration to the attenuation characteristics of the amount of light of the pulsed light in the object in order to obtain a photoacoustic wave image having a uniform brightness level regardless of the measured depth.

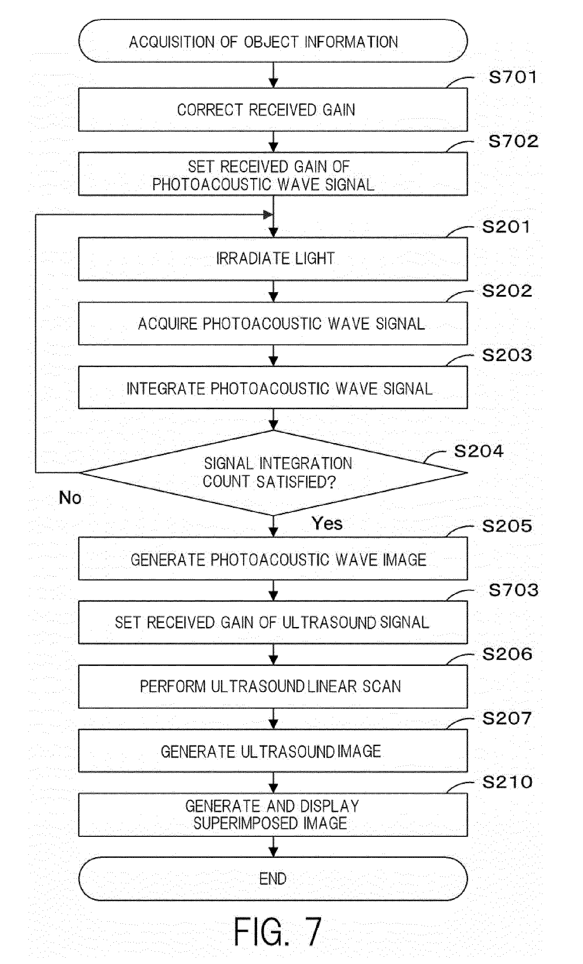

FIG. 7 is a flowchart showing the flow of acquiring object information in this embodiment. The flowchart of FIG. 7 is implemented when the user commands the start of acquisition of object information via the operation unit 110. Note that the differences in comparison to the flowchart of FIG. 2 are step S701, step S702, and step S703.

In step S701, the control processor 109 reads the current setting value of the corrector 303 for the photoacoustic wave image and the corrector 307 for the ultrasound image.

In step S702, the control processor 109 generates a received gain table for the photoacoustic wave signals which stores the received gain value for each received sample according to the setting of the corrector 303 for the photoacoustic wave image, and sets such received gain table in the signal reception unit 105. The received gain table is similar to the correction value curve shown in FIG. 4B and is, for example, sequence data in which the vertical axis is the number of received samples along the time series of the signal reception unit, and the horizontal axis is the amplification gain in the signal amplifier or the A/D converter of the signal processor. The signal reception unit 105 performs TGC according to this received gain table.

In step S201 to step S204, as with Embodiment 1, acquisition of photoacoustic wave signals that satisfy the signal integration count designated in advance is performed. However, in step S202 in this embodiment, the photoacoustic wave signals that were subject to TGC are obtained according to the received gain table set in step S701.

In step S205, the image component 111 generates a photoacoustic wave image based on the signals performed in TGC.

In step S703, the control processor 109 generates a received gain table for ultrasound signals according to the setting of the corrector 307 for the ultrasound image, and sets such received gain table in the signal reception unit 105.

In step S206, the ultrasound transmission controller 107 performs a linear scan based on the transmission of ultrasound beams and the reception of ultrasound echoes. Note that, upon the reception of the ultrasound echoes, the signal reception unit 105 performs TGC according to the received gain table set in step S703.

In step S207, the ultrasound signal processor 108 generates the B-mode image by performing phasing addition processing, logarithmic compression, filter processing and the like on the digital signals of the ultrasound waves that were obtained in step S206 after performing TGC.

In step S210, the image component 111 uses the photoacoustic wave image and the ultrasound image generated as a result of step S205 and step S207 to configure a superimposed image, and displays the superimposed image on the display unit 112.

According to the object information acquiring apparatus configured as described above, it is possible to provide a function of performing received gain control upon the reception of signals on the corrector 303 for the photoacoustic wave image and the corrector 307 for the ultrasound image in a single acquisition operation of object information. Consequently, the photoacoustic wave image and the ultrasound image that were subject to TGC can be acquired collectively. According to this embodiment, the first corrector of the present invention includes a control processor for controlling the TGC processing to be performed on the photoacoustic signals, and a signal reception unit for executing such processing. Similarly, the second corrector also includes a TGC function of the control processor to be performed on the ultrasound signals and an amplification function of the signal reception unit.

Note that the series of processing in the flowchart of FIG. 7 were explained as being completed with the single acquisition of object information in response to a single acquisition command. As a result of the apparatus repeatedly performing this flowchart at given intervals, it is possible to follow, within a given period of time, the correction operations that were continued by the user. As a result of providing separate brightness correctors for the photoacoustic wave image and the ultrasound image having different attenuation characteristics and responding in real-time, within a given period of time, to the user's correction operations, it is possible to further assist the user's operations for adjusting TGC that is suitable for observation.

<Embodiment 4>

Embodiment 4 of the present invention is now explained with reference to the drawings. The correction method of objection information in this embodiment is to return, to the initial value, the correction of the brightness value or the correction setting of the reception gain which were performed in the depth direction of the photoacoustic image and the ultrasound image in each of the foregoing embodiments.

Note that the apparatus configuration of the object information acquiring apparatus in this embodiment is the same as the apparatus configuration shown in FIG. 1, and the explanation thereof is omitted. Moreover, since the flowchart related to the acquisition of object information is also the same as the flowchart shown in FIG. 3, the UI to be presented to the user can also be implemented in the modes explained with reference to FIG. 3 and FIG. 4.

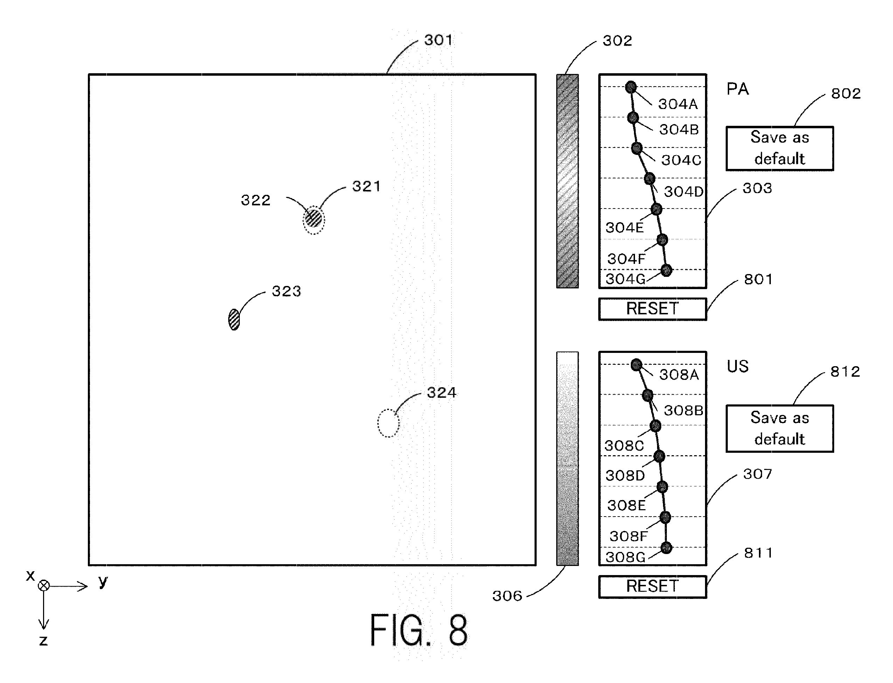

FIG. 8 is a conceptual diagram explaining the corrector of object information in this embodiment. The corrector in this embodiment is provided as a UI that is displayed on the display unit 112. Note that the application of this invention is not limited to the configuration based on the UI of software, and the corrector function may also be provided with hardware such as a slide bar or a dial.

A new function is disposed in addition to the UI of FIG. 2 in Embodiment 1. Reference numeral 801 shows a reset button of the correction value of the photoacoustic wave image, and reference numeral 802 shows a default setup button for updating the default correction value of the photoacoustic wave image that is retained in the storage unit 113. In this embodiment, the reset button 801 and the default setup button 802, together with the corrector 303, are the UI corresponding to the photoacoustic wave corrector 110A.

When the default setup button 802 is pressed, a default setup button event is notified to the control processor 109. The control processor 109 that received the notice stores and retains, in the storage unit 113, the setting of the correction control points 304A to 304G which have been set up to the time that the default setup button 802 was pressed.

Moreover, reference numeral 811 shows a reset button of the correction value of the ultrasound image, and reference numeral 812 shows a setup button for updating the default correction value of the ultrasound image that is retained in the storage unit 113. In this embodiment, the reset button 811 and the default setup button 812, together with the corrector 307, are the UI corresponding to the ultrasound wave corrector 110B.

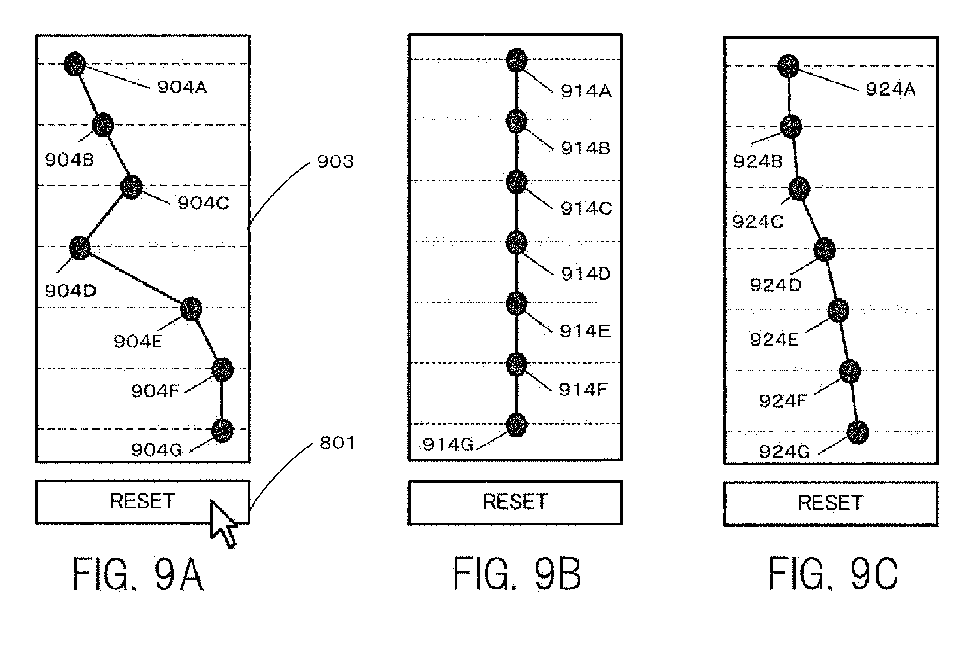

The correction method of object information in this embodiment is now explained with reference to FIG. 9. FIG. 9A shows the state of the correction value control points resulting from the user's free operation. FIG. 9B and FIG. 9C respectively show the results upon applying the correction method of this embodiment. Note that the corrector in Embodiment 1 is provided as the UI displayed on the display unit 112. However, the application of the present invention is not limited to the configuration based on the UI of software, and the corrector function may also be provided with hardware such as buttons.

Reference numeral 903 shows a state where the correction control points 904A to 904G have been disposed according to the user's operation of the corrector 303 of the photoacoustic wave image.

When the user presses the reset button 801 from the foregoing state, a reset button event is notified to the control processor 109. The control processor 109 that received the foregoing notice reads the correction default value retained in the storage unit 113, and reflects the read correction default value in the corrector 303.

FIG. 9B shows a state (914A to 914G) in which all correction control points were uniformly set to an intermediate value as a result of the user pressing the reset button 801. This, for example, is the state where the setting has been reset to the factory default setting.

FIG. 9C shows a state (924A to 924G) in which the correction control points have been set to a correction default value at a previous updated point in time as a result of the user pressing the reset button 801.

As a result of the user pressing the reset button, a value change event occurs since the correction control points will move, and it is thereby possible to reflect the corrections according to the flowchart (FIG. 5) for correcting the object information in Embodiment 1 or the flow (FIG. 7) for acquiring the object information in Embodiment 3.

Moreover, as a result of the user performing similar operations by using the corrector 307 for correcting ultrasound waves and reset button 811, the correction based on the reset button 811 can be applied to the ultrasound image.

In the object information acquiring apparatus configured as described above, it is possible to collectively set the default value; that is, reset the setting, after the initial correction or after the user operates the individual correction control points on the photoacoustic wave image and the ultrasound image. It is thereby possible to eliminate a complicated user operation.

<Embodiment 5>

Moreover, the object of the present invention can also be achieved based on the following means. In other words, the storage medium storing the program codes of the software for realizing the functions of the embodiments described above is supplied to the system or the apparatus. In addition, the computer (CPU or the like) of that system or apparatus reads and executes the program codes stored in the storage medium. In the foregoing case, the program codes read from the storage medium will directly realize the functions of the foregoing embodiments, and the storage medium storing such program codes will configure the present invention.

Moreover, as a result of executing the program codes read by the computer, the operating system (OS) or the like running on that computer will perform a part or all of the actual processing based on the instructions of the program code. It goes without saying that the present invention covers cases where the functions of the foregoing embodiments are realized with such processing.

In addition, let it be assumed that the program codes read from the storage medium are written into a memory of a function enhancement card inserted into the computer or a function enhancement unit connected to the computer. It goes without saying that the present invention covers cases where the functions of the foregoing embodiments are realized by, fir example, the CPU of the function enhancement card or the function enhancement unit subsequently performing a part or all of the actual processing based on the instructions of such program codes.

When the present invention is applied to the foregoing storage medium, the storage medium will store the program codes corresponding to the previously explained flowcharts.

Since it would be easy for a person skilled in the art to suitably combine the various technologies described in each of the foregoing embodiments and configure a new system, systems based on such various combinations are also covered by the scope of the present invention.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An object information acquiring apparatus is used that include a probe; a signal processor that generates a first image based on photoacoustic waves, which are generated from an object irradiated with light and which are received by the probe, and that generates a second image based on ultrasound waves, which are received by the probe and then transmitted to the object and reflected off the object; a controller that displays the first image and the second image on a display unit; a first corrector that corrects brightness of the first image; and a second corrector that corrects brightness of the second image independently from the first corrector.

**Claims**

1.  An object information acquiring apparatus, comprising:

    a probe;
    a signal processor configured to generate a first image based on photoacoustic waves, which are generated from an object irradiated with light and which are received by the probe, and generate a second image based on ultrasound waves, which are transmitted to the object and reflected by the object and then received by the probe;
    a controller configured to display the first image and the second image on a display unit;
    a first corrector configured to correct brightness of the first image; and
    a second corrector configured to correct brightness of the second image independently from the first corrector.

2.  The object information acquiring apparatus according to claim 1, wherein
    the first corrector corrects the brightness of the first image according to a distance from the probe.

3.  The object information acquiring apparatus according to claim 1, wherein
    the first corrector corrects the brightness of the first image according to a distance from a surface of the object.

4.  The object information acquiring apparatus according to any one of claims 1 to 3, wherein
    the second corrector corrects the brightness of the second image according to a distance from the probe.

5.  The object information acquiring apparatus according to any one of claims 1 to 4, wherein
    the controller displays a superimposed image between the first image and the second image on the display unit.

6.  The object information acquiring apparatus according to any one of claims 1 to 4, wherein
    the controller displays the first image and the second image side by side on the display unit.

7.  The object information acquiring apparatus according to any one of claims 1 to 6, wherein
    the first corrector and the second corrector include an operation unit for adjusting a correction value of the brightness of the first image and the second image.

8.  The object information acquiring apparatus according to claim 7, wherein
    the controller displays a graphical user interface indicating the correction value on the display unit, and the operation unit operates the correction value based on the graphical user interface indicating the correction value displayed on the display unit.

9.  The object information acquiring apparatus according to claim 8, wherein
    the controller displays, on the display unit, the graphical user interface indicating the correction value side by side with the first image and the second image.

10. The object information acquiring apparatus according to any one of claims 1 to 9, further comprising:

    a brightness distribution calculation unit configured to obtain a brightness distribution of at least either the first image or the second image, wherein
    when a first brightness distribution indicating a brightness distribution of the first image is obtained by the brightness distribution calculation unit, the first corrector performs correction based on the first brightness distribution, and when a second brightness distribution indicating a brightness distribution of the second image is obtained by the brightness distribution calculation unit, the second corrector performs correction based on the second brightness distribution.

11. The object information acquiring apparatus according to claim 10, wherein
    when the first brightness distribution is obtained by the brightness distribution calculation unit, the first corrector determines a first reference as the brightness included in the first brightness distribution, and corrects the brightness of the first image based on the first reference, and
    when the second brightness distribution is obtained by the brightness distribution calculation unit, the second corrector determines a second reference as the brightness included in the second brightness distribution, and corrects the brightness of the second image based on the second reference.

**12.** The object information acquiring apparatus according to any one of claims 1 to 11, wherein
the signal processor amplifies a first electric signal that is obtained by the probe receiving the photoacoustic waves, and also amplifies a second electric signal that is obtained by the probe receiving the ultrasound waves,
the first corrector controls an amplification gain of the first electric signal according to a period from the time when the photoacoustic waves are generated until when the waves reach the probe, and
the second corrector controls an amplification gain of the second electric signal according to a period from the time when the ultrasound waves are reflected until when the waves reach the probe.

**13.** The object information acquiring apparatus according to claim 12, wherein
the first corrector controls the amplification gain of the first electric signal according to attenuation characteristics, in the object, of the light with which the object has been irradiated, and attenuation characteristics of the photoacoustic waves generated in the object.

**14.** The object information acquiring apparatus according to claim 12, wherein
the second corrector controls the amplification gain of the second electric signal according to attenuation characteristics of the ultrasound waves reflected in the object.

FIG. 1

START ACQUISITION OF
OBJECT INFORMATION

S201 — IRRADIATE LIGHT

S202 — ACQUIRE PHOTOACOUSTIC WAVE SIGNAL

S203 — INTEGRATE PHOTOACOUSTIC WAVE SIGNAL

S204 — SIGNAL INTEGRATION COUNT SATISFIED?

No

Yes

S205 — GENERATE PHOTOACOUSTIC WAVE IMAGE

S206 — PERFORM ULTRASOUND LINEAR SCAN

S207 — GENERATE ULTRASOUND IMAGE

S208 — APPLY INITIAL CORRECTION TO PHOTOACOUSTIC WAVE IMAGE

S209 — APPLY INITIAL CORRECTION TO ULTRASOUND IMAGE

S210 — GENERATE AND DISPLAY SUPERIMPOSED IMAGE

S211 — CORRECT SUPERIMPOSED IMAGE

S212 — IS IMAGE OBSERVATION COMPLETE?

No

Yes

END

FIG. 2

FIG. 3

EP 2 749 227 A1

A

B

304A

304B

411C

401

412C

C

304D

D

E

304E

F

304F

G

304G

303

x

y

z

Min

Max

A

B

C

D

E

F

G

Z
(DEPTH DIRECTION)

# FIG. 4A

# FIG. 4B

IMAGE CORRECTION
(BRIGHTNESS CORRECTION)

VALUE CHANGE EVENT
OF BRIGHTNESS CONTROL
NOTIFIED? — S501

No

Yes

CALCULATE BRIGHTNESS CORRECTION — S502

REFLECT BRIGHTNESS CORRECTION VALUE
IN TARGET IMAGE — S503

UPDATE DISPLAY OF SUPERIMPOSED IMAGE — S504

END

FIG. 5

APPLICATION OF INITIAL CORRECTION TO IMAGE

↓ S601

APPLICATION OF INITIAL CORRECTION TO IMAGE

↓ S602

CALCULATE DISTRIBUTION OF
DEPTH DIRECTION BRIGHTNESS LEVEL

↓ S603

CALCULATE AND SET INITIAL CORRECTION VALUE

↓ S604

APPLY INITIAL CORRECTION TO IMAGE

↓ S605

DISPLAY IMAGE

↓

END

# FIG. 6

ACQUISITION OF OBJECT INFORMATION

S701
CORRECT RECEIVED GAIN

S702
SET RECEIVED GAIN OF
PHOTOACOUSTIC WAVE SIGNAL

S201
IRRADIATE LIGHT

S202
ACQUIRE PHOTOACOUSTIC WAVE SIGNAL

S203
INTEGRATE PHOTOACOUSTIC WAVE SIGNAL

S204
SIGNAL INTEGRATION
COUNT SATISFIED?

No

Yes

S205
GENERATE PHOTOACOUSTIC WAVE IMAGE

S703
SET RECEIVED GAIN OF ULTRASOUND SIGNAL

S206
PERFORM ULTRASOUND LINEAR SCAN

S207
GENERATE ULTRASOUND IMAGE

S210
GENERATE AND DISPLAY
SUPERIMPOSED IMAGE

END

FIG. 7

FIG. 8

FIG. 9A

FIG. 9B

FIG. 9C

Europäisches
Patentamt

European
Patent Office

Office européen
des brevets

## EUROPEAN SEARCH REPORT

Application Number

EP 13 19 6355

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/319743 A1 (SATOH YOSHIAKI [JP]) 29 December 2011 (2011-12-29) | 1-6,12, 14 | INV. A61B8/00 |
| Y | * paragraphs [0056] - [0059], [0076] - [0090]; claims 1-20; figures 1-7 * | 7-11,13 | A61B8/08 A61B5/00 |
| | ----- | | |
| X | US 2011/245652 A1 (OISHI TAKUJI [JP]) 6 October 2011 (2011-10-06) * paragraphs [0030] - [0048]; claims 1-9; figures 1-8 * | 1,10-14 | |
| | ----- | | |
| Y | US 2011/054320 A1 (NISHIHARA KURAMITSU [JP] ET AL) 3 March 2011 (2011-03-03) * paragraphs [0003] - [0012], [0015], [0022] - [0035], [0045]; figures 1-3 * | 7-11 | |
| | ----- | | |
| Y | US 2012/243369 A1 (SUDO YOSHIAKI [JP] ET AL) 27 September 2012 (2012-09-27) * paragraphs [0040] - [0046], [0053] - [0062]; figures 1-3, 57 * | 13 | |
| | ----- | | |

TECHNICAL FIELDS
SEARCHED (IPC)

A61B

The present search report has been drawn up for all claims

| Place of search | Date of completion of the search | Examiner |
|---|---|---|
| Munich | 27 March 2014 | Apostol, Simona |

EPO FORM 1503 03.82 (P04C01)

**ANNEX TO THE EUROPEAN SEARCH REPORT
ON EUROPEAN PATENT APPLICATION NO.**

EP 13 19 6355

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2014

| Patent document cited in search report | | Publication date | Patent family member(s) | | Publication date |
|---|---|---|---|---|---|
| US 2011319743 | A1 | 29-12-2011 | CN | 102293666 A | 28-12-2011 |
| | | | JP | 2012005624 A | 12-01-2012 |
| | | | US | 2011319743 A1 | 29-12-2011 |
| US 2011245652 | A1 | 06-10-2011 | JP | 2011212254 A | 27-10-2011 |
| | | | US | 2011245652 A1 | 06-10-2011 |
| US 2011054320 | A1 | 03-03-2011 | CN | 101999905 A | 06-04-2011 |
| | | | JP | 2011045486 A | 10-03-2011 |
| | | | US | 2011054320 A1 | 03-03-2011 |
| US 2012243369 | A1 | 27-09-2012 | JP | 2011152273 A | 11-08-2011 |
| | | | US | 2012243369 A1 | 27-09-2012 |
| | | | WO | 2011093069 A1 | 04-08-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4448189 B

- JP 2010015535 A

**Non-patent literature cited in the description**

- *JOURNAL OF BIOMEDICAL OPTICS,* July 1996, vol. 1 (3), 330-334